# EUROPEAN PATENT SPECIFICATION

(11) **EP 3 019 460 B1**
(45) Date of publication and mention of the grant of the patent: **30.01.2019**
(21) Application number: 14737234.6
(22) Date of filing: 08.07.2014
(51) Int. Cl.: C07C 29/14, C07C 33/02

(54) **SELECTIVE TRANSFER HYDROGENATION OF CITRAL OR ETHYL CITRAL**
SELEKTIVE TRANSFERHYDRIERUNG VON CITRAL ODER ETHYLCITRAL
HYDROGÉNATION SÉLÉCTIVE PAR TRANSFERT DU CITRAL OU DU CITRAL D'ÉTHYLE

(30) Priority: 08.07.2013 EP 13175592
(43) Date of publication of application: 18.05.2016
(73) Proprietor: DSM IP Assets B.V., 6411 TE Heerlen (NL)
(72) Inventor: BONRATH, Werner, 4002 Basel (CH); MEDLOCK, Jonathan, Alan, 4002 Basel (CH); WÜSTENBERG, Bettina, 4002 Basel (CH); SCHÜTZ, Jan, 4002 Basel (CH); NETSCHER, Thomas, 4002 Basel (CH)
(74) Representative: Kurt, Manfred
(86) International application number: PCT/EP2014/064564
(87) International publication number: WO 2015/004116

(56) References cited:
- XIAOFENG WU ET AL: "A Versatile Iridium Catalyst for Aldehyde Reduction in Water", CHEMSUSCHEM, WILEY - VCH VERLAG GMBH & CO. KGAA, DE, IT, vol. 1, no. 1-2, 22 February 2008 (2008-02-22), pages 71-74, XP008164843, ISSN: 1864-5631, DOI: 10.1002/CSSC.200700086 [retrieved on 2008-01-02]
- FLEISCHER ET AL.: "General and Highly Efficient Iron-Catalyzed Hydrogenation of Aldehydes, Ketones and alpha,beta-Unsaturated Aldehydes", ANGEW. CHEM. INT. ED., vol. 52, 3 May 2013 (2013-05-03), pages 5120-5124, XP002720159,

## Description

The present invention relates to a selective transfer hydrogenation of citral to geraniol/nerol and ethyl citral to ethyl geraniol/ethyl nerol in the presence of a specific amino-amide-transition-metal catalyst and a H₂ donor.

Citral (IUPAC: 3,7-dimethyl-2,6-octadienal) is the compound of formula (Ia)

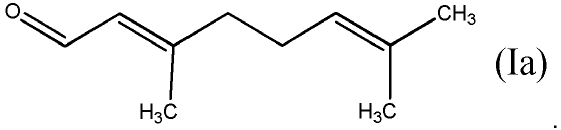

Ethyl citral (IUPAC: 3,7-dimethyl-2,6-nonadienal) is the compound of formula (Ib)

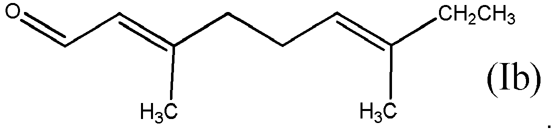

The chemical formulae as drawn above cover all isomeric configurations these compounds can have.

Citral is usually a mixture of the E- and the Z-isomer. The E-isomer is known as geranial or citral A (compound of formula (Ia'): 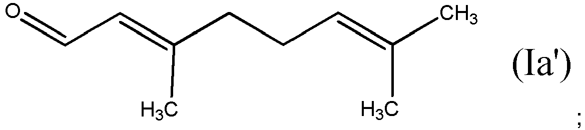 and the Z-isomer is known as neral or citral B (compound of formula (Ia"):

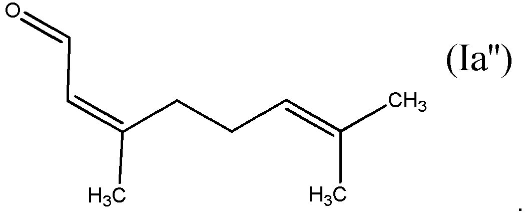

The same applies for the ethyl citral. It also exists in different isomeric configurations due to both double bonds. Each carbon-carbon double bond can independently have the E or Z configuration.

Citral can be extracted from various plants such as for example lemon myrtle, Litsea citrata, Litsea cubeba, lemongrass, lemon tea-tree, Ocimum gratissimum, Lindera citriodora, Calypranthes parriculata, petitgrain, lemon verbena, lemon ironbark (26%), lemon balm, lime, lemon, and orange.
It also possible and more common to chemically synthesize citral. Citral is available commercially from various companies.

The goal of the present invention was to find an improved way to provide geraniol/nerol (compound of formula (IIa)) and ethyl geraniol/ethyl nerol (compound of formula (IIb)), 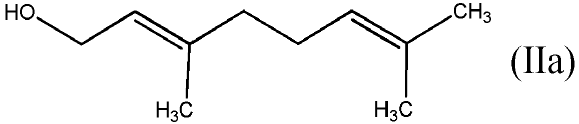 and 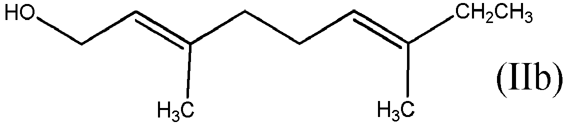 which are the selectively hydrogenated products of citral and ethyl citral respectively.

In nature geraniol, which is the compound of formula (IIa') 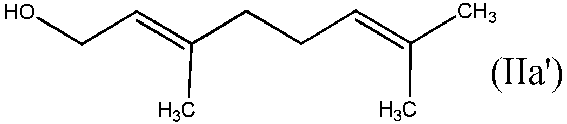 can be found in rose oil, palmarosa oil, and citronella oil (Java type). It also occurs in small quantities in geranium, lemon, and many other essential oils. It appears as a clear to pale-yellow oil that is insoluble in water, but soluble in most common organic solvents. It has a rose-like scent and is commonly used in perfumes. It is used in flavors such as peach, raspberry, grapefruit, red apple, plum, lime, orange, lemon, watermelon, pineapple, and blueberry.

In nature nerol, which is the compound of formula (IIa") 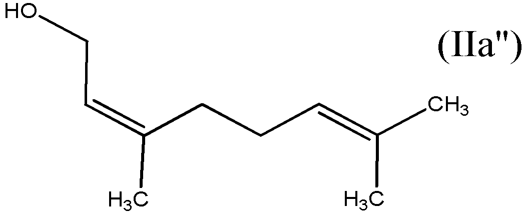 can be found in many essential oils such as lemongrass and hops. It was originally isolated from neroli oil, hence its name. This colourless liquid is used in perfumery. Like geraniol, nerol has a sweet rose odor but it is considered to be fresher.

Geraniol and nerol (as well as the mixture of these compounds) are very important compounds in fragrance and flavour applications. Therefore, there is always a need for an improved way for obtaining these compounds.

Some specific transfer hydrogenations of citral are known from the prior art, but they have some disadvantages in regard to selectivity and/or conversion and/or yield and/or reaction conditions.

Surprisingly we found that the using specific kind of catalysts and reduction agents it is possible to hydrogenate citral selectively by a transfer hydrogenation reaction whereas the selectivity, the conversion as well as the yield of the hydrogenation are excellent.

The present invention relates to a transfer hydrogenation (TH) of a compound of formula (I) 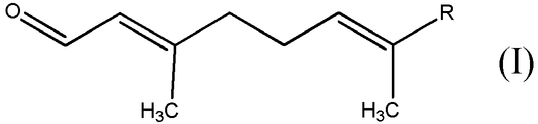 to a compound of formula (II) 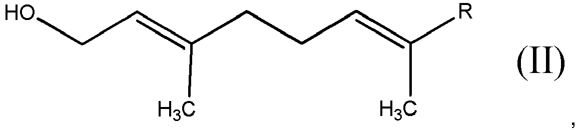 wherein R signifies -CH₃ or -CH₂CH₃,
characterized in that the hydrogenation is carried out in the presence of
a) an amino-amide-transition-metal catalyst of formula (III)

   MX[Z-(-H)][Y] (III)

   wherein
   - M: is chosen from the group consisting of Ir, Rh and Ru, and
   - X: signifies H or a halogen atom, and
   - Y: is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
   - Z: signifies a group of formula (IV) or (V) 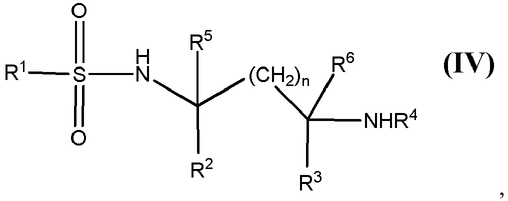 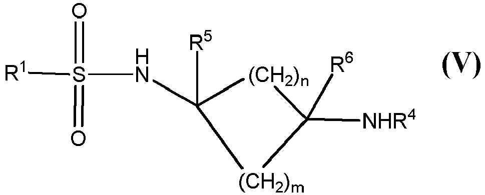
   wherein
   R¹ signifies C₁-C₆-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
   R² and R³ each independently signify hydrogen, C₁-C₂-alkyl, C₄-C₇-cycloalkyl, or aryl which may be substituted, and
   R⁴ signifies hydrogen or C₁-C₂-alkyl, and
   R⁵ signifies hydrogen or C₁-C₂-alkyl, and
   R⁶ signifies hydrogen or C₁-C₂-alkyl, and
   n signifies 0,1,2 or 3, and
   m signifies 0,1,2 or 3, and
   with the proviso that in formula (V) the sum of n and m is 3 or 4, and
b) a H₂ donor, and
   wherein the hydrogenation is carried out by using at least one solvent which also serves as hydrogen donor.

The reduction of aldehydes in water under H₂ or with formate in the presence of tosylated Ir catalysts is already described by Xiaofeng Wu et al. in ChemSusChem 2008, 1, 71-74 ("A Versatile Iridium Catalyst for Aldehyde Reduction in Water").

A preferred transfer hydrogenation (TH¹) is (TH), characterized in that the hydrogenation is carried out in the absence of water.
This (*"by the absence of water"*) means that water is not added intentionally to the reaction solution. The water content is usually below 3 weight-% (wt-%), preferably below 2 wt-%, based on the total weight of the reaction solution.

The selectivity, the yield and the conversion of the transfer hydrogenation according to the present invention are excellent.

The monosulphonylated diamine is present in the complex as a monoanion and is accordingly denoted in formula III as "Z-(-H)".

By the term "amino-amide transition metal catalyst" it is meant that the transition metal is complexed to Z via the amine and the amide of Z.

Transfer hydrogenation is the addition of hydrogen to a compound (molecule) from a source other than gaseous H₂.
The use of H₂ gas has some issues with the handling of the explosive gas in regard to safety and in regard to the apparatus needed.

Instead of H₂ gas a H₂ donor system is used for transfer hydrogenation.

The transfer hydrogenation according to the present invention is carried out by using at least one solvent which also serves as hydrogen donor.

Therefore, the present invention is directed towards a transfer hydrogenation (TH²), which is (TH) or (TH¹), wherein the hydrogenation is carried out by using at least one solvent which also serves as hydrogen donor.

As the hydrogen donor and at the same time the solvent there can be used especially alcohols, preferably secondary alcohols, e.g. isopropanol.

Therefore, a further embodiment of the present invention is a transfer hydrogenation (TH³), which is (TH), (TH¹) or (TH²), wherein the hydrogenation is carried out by using isopropanol as hydrogen donor and solvent.

In the scope of the present invention the term "substituted with one or more fluorine atoms", otherwise expressed as "mono- or multiply-fluorinated", means having at least one fluorine substituent to as many fluorines as the alkyl group so modified is capable of accepting; however one to five fluorines is preferred.

In the scope of the present invention the term "alkyl" embraces straight-chain or branched alkyl groups with 1 to 6 carbon atoms, preferably 1 to 4 carbon atoms. Methyl, ethyl, propyl, isopropyl, n-butyl, isobutyl and tert.-butyl, are examples such alkyl groups. Trifluromethyl, 2,2,2-trifluoroethyl and pentafluoroethyl are examples of mono- or multiply-fluorinated alkyl groups.

The term "alkenyl" embraces straight-chain or branched alkenyl groups with 2 to 6 carbon atoms, e.g. allyl, 2-butenyl and 3-butenyl.

The term "alkynyl" signifies a straight-chain or branched alkynyl group with one triple bond and 2 to 6 carbon atoms, e.g. propynyl and butynyl.

The term "cycloalkyl" signifies a 4- to 7-membered alicyclic group, namely cyclobutyl, cyclopentyl, cyclohexyl or cycloheptyl, of which cyclopentyl and cyclohexyl are preferred.

The term "unsubstituted or substituted aryl" or equally "aryl which may be substituted" or "optionally mono- or multiply-substituted aryl" preferably embraces a phenyl or naphthyl group, which can be unsubstituted, mono-substituted or multiply-substituted. As substitutents there come into consideration e.g. phenyl, halogen and straight-chain and branched alkyl and alkoxy groups with in each case 1 to 5 carbon atoms, whereby the multiply-substituted phenyl or naphthyl groups can have the same or different substituents. Of the alkyl and alkoxy groups the methyl and, respectively, the methoxy group is preferred. Examples of optionally substituted aryl groups are phenyl, chloro-, bromo- and fluorophenyl, tolyl, anisyl, as well as naphthyl. The place of the substitution can be at any position of the aromatic ring.

The term "heteroaryl" embraces 5- or 6-membered heterocyclic groups featuring O, S or N as a ring member, i.e. heteroatom, such as, for example, furyl, thienyl, benzofuryl, dibenzofuryl, xanthenyl, pyrrolyl and pyridinyl. The heterocyclic groups featuring O as the heteroatom are especially preferred.

A preferred embodiment of the present invention relates to a transfer hydrogenation of citral or ethyl citral, wherein the catalyst of formula (III) is as defined above.

Therefore, a further embodiment of the present invention is a transfer hydrogenation (TH⁴), which is (TH), (TH¹), (TH²) or (TH³), wherein the catalyst is as defined above.

A more preferred embodiment of the present invention relates to a transfer hydrogenation of citral or ethyl citral, wherein the catalyst of formula (III)
- M: is chosen from the group consisting of Ir, Rh and Ru, preferably Ru, and
- X: signifies H or Cl, and
- Y: is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
- Z: signifies a group of formula (IV) 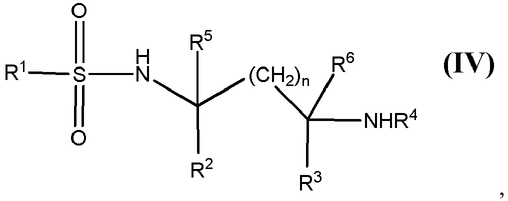
wherein
R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
R² and R³ each independently signify hydrogen, C₁-C₂-alkyl, C₄-C₇-cycloalkyl, or aryl which may be substituted, and
R⁴ signifies hydrogen or C₁-C₂-alkyl, and
R⁵ signifies hydrogen or C₁-C₂-alkyl, and
R⁶ signifies hydrogen or C₁-C₂-alkyl, and
n signifies 0,1,2 or 3.

Therefore, a further embodiment of the present invention is a transfer hydrogenation (TH⁵), which is (TH), (TH¹), (TH²), (TH³) or (TH⁴), wherein
within the catalyst of formula (III)
- M: is chosen from the group consisting of Ir, Rh and Ru, preferably Ru
- X: signifies H or Cl, and
- Y: is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
- Z: signifies a group of formula (IV) 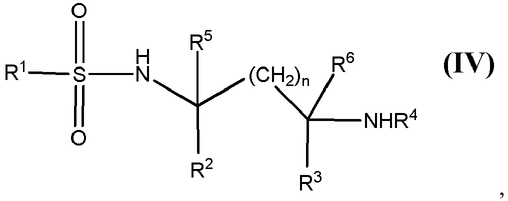
wherein
R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
R² and R³ each independently signify hydrogen, C₁-C₂-alkyl, C₄-C₇-cycloalkyl, or aryl which may be substituted, and
R⁴ signifies hydrogen or C₁-C₂-alkyl, and
R⁵ signifies hydrogen or C₁-C₂-alkyl, and
R⁶ signifies hydrogen or C₁-C₂-alkyl, and
n signifies 0,1,2 or 3.

Furthermore, the present invention also relates to preferred transfer hydrogenations of citral or ethyl citral, wherein the catalyst is of formula (III') 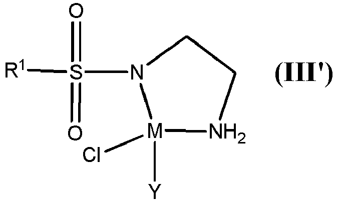 wherein
M is Ru, Rh or Ir, preferably Ru, and
Y is an aromatic ligand chosen from the group consisting of 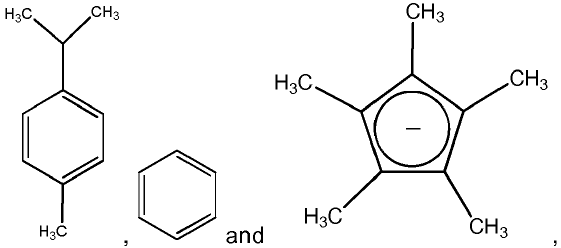 and
R¹ is -CH₃,

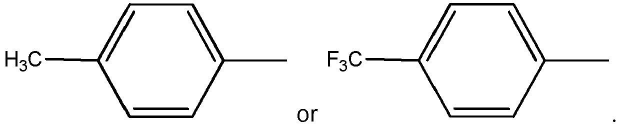

Therefore, a further embodiment of the present invention is a transfer hydrogenation (TH⁶), which is (TH), (TH¹), (TH²), (TH³), (TH⁴) or (TH⁵), wherein
the catalyst is of formula (III') 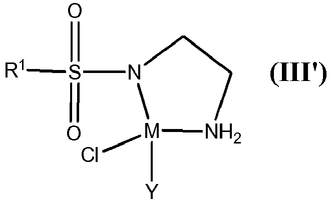 wherein
M is Ru, Rh or Ir, preferably Ru, and
Y is an aromatic ligand chosen from the group consisting of 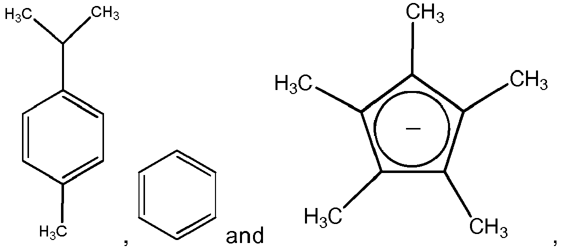 and
R¹ is -CH₃,

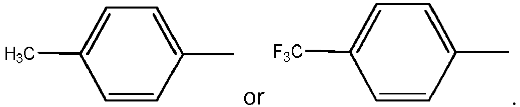

Preferred transfer hydrogenations according to the present invention are such wherein the following catalysts are used: 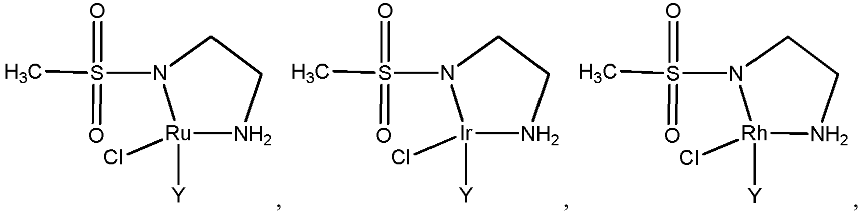 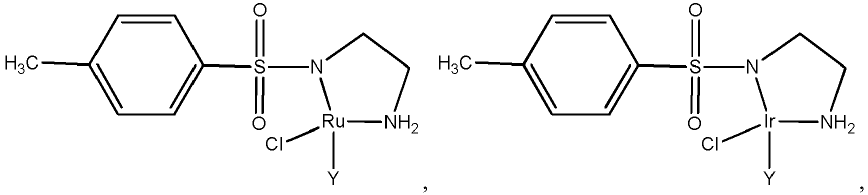 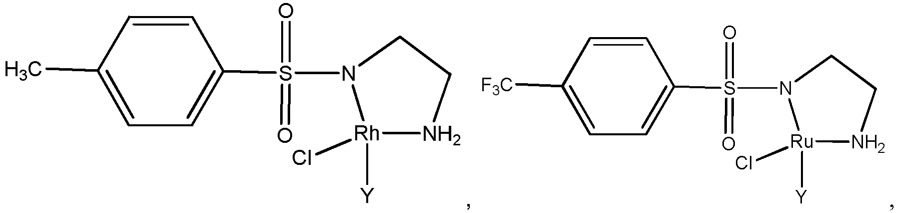 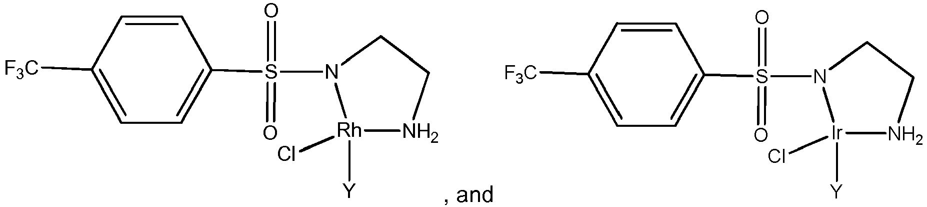 wherein Y is an aromatic ligand chosen from the group consisting of

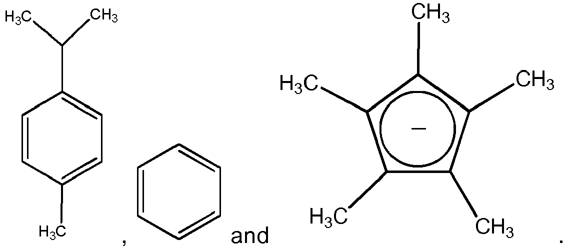

Therefore, a further embodiment of the present invention is a transfer hydrogenation (TH⁷), which is (TH), (TH¹), (TH²), (TH³), (TH⁴), (TH⁵) or (TH⁶), wherein the catalyst is chosen from the group consisting of 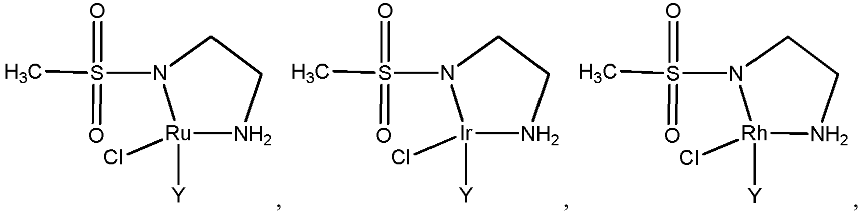 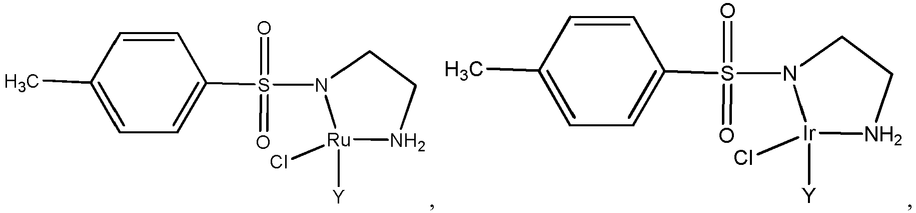 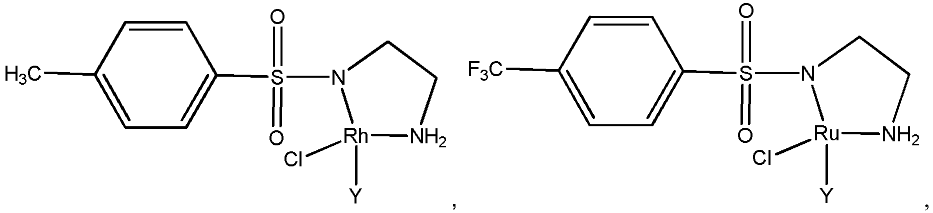 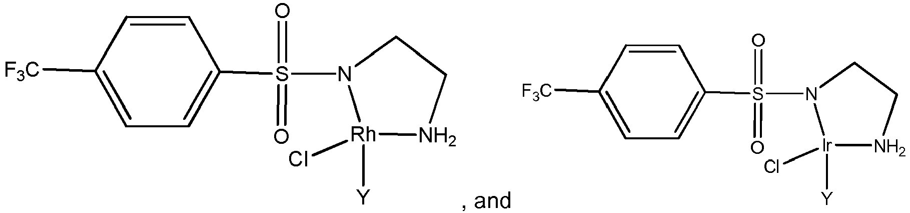 wherein Y is an aromatic ligand chosen from the group consisting of

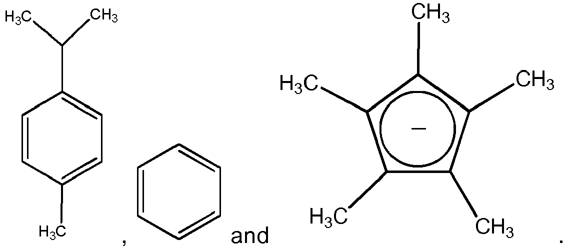

More preferred transfer hydrogenations according to the present invention are such wherein the following catalysts are used: 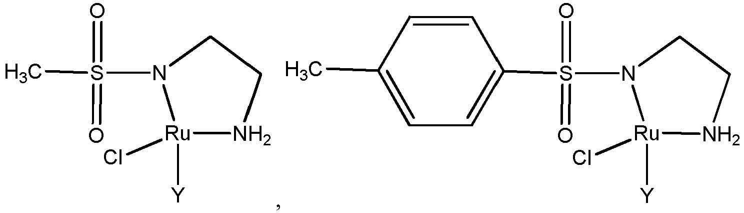 and 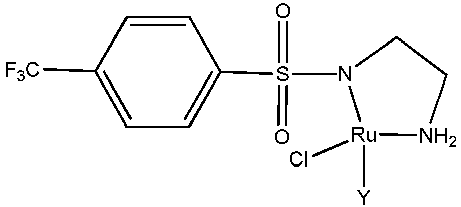 wherein Y is an aromatic ligand chosen from the group consisting of

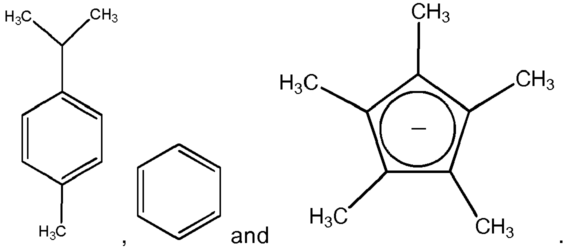

Therefore, a further embodiment of the present invention is a transfer hydrogenation (TH⁸), which is (TH), (TH¹), (TH²), (TH³), (TH⁴), (TH⁵), (TH⁶) or (TH⁷), wherein the catalyst is chosen from the group consisting of 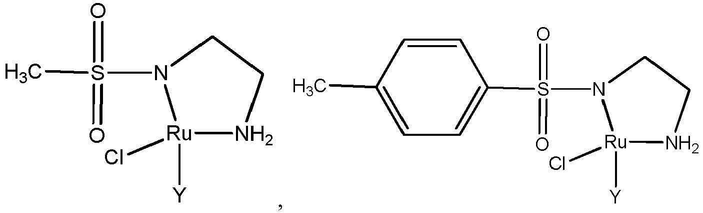 and 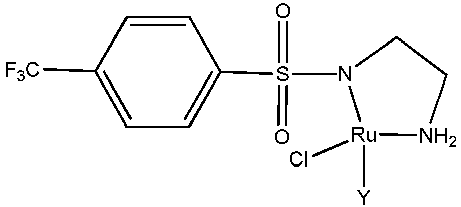 wherein Y is an aromatic ligand chosen from the group consisting of

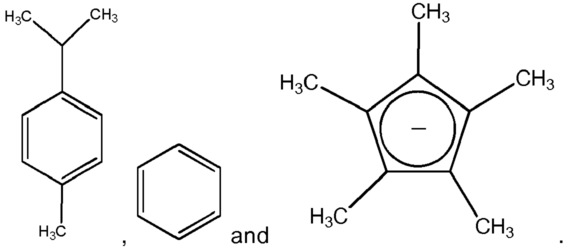

Preferably the catalyst is prepared in situ starting from a metal precursor complex and various ligands:
0.5 Mol [M(Y)X₂]₂ and 1 Mol Z, which is as stated above represented by formula (IV) or (V) 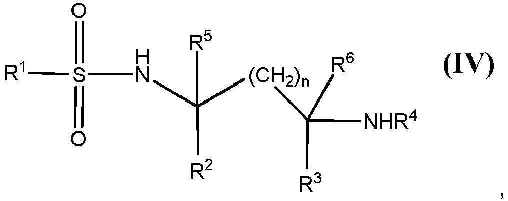 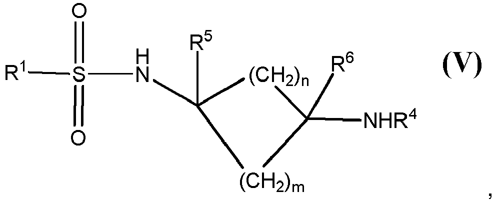 wherein all the substituents have the same meanings as defined above, are mixed to form the catalyst.
All the substituents have the same meanings as defined above as well as the same preferences.

The reaction temperature of the process of production of the catalyst (compound of formula (III)) is usually 0 - 100°C. The reactants are usually mixed for a period of time and then all the reactants for the selective transfer hydrogenation of the present invention are added and the necessary reaction conditions of the hydrogenation are applied.

The selective transfer hydrogenation reaction is usually carried out at a temperature of 0 - 100 °C, preferably 25 - 90°C.

Therefore, a further embodiment of the present invention is a transfer hydrogenation (TH⁹), which is (TH), (TH¹), (TH²), (TH³), (TH⁴), (TH⁵), (TH⁶), (TH⁷) or (TH⁸), wherein the transfer hydrogenation reaction is carried out at a temperature of 0 - 100 °C, preferably 25 - 90°C.

The selective transfer hydrogenation reaction is usually carried out at a normal (ambient) pressure. It could also been carried out at elevated and preferably at reduced pressure. Usually the transfer hydrogenation according to the present invention is carried out at a pressure of 50 - 1000 mbar.

Therefore, a further embodiment of the present invention is a transfer hydrogenation (TH¹⁰), which is (TH), (TH¹), (TH²), (TH³), (TH⁴), (TH⁵), (TH⁶), (TH⁷), (TH⁸) or (TH⁹), wherein the transfer hydrogenation reaction is carried out at a pressure of 50 - 1000 mbar.

The substrate to catalyst ratio (s/c ratio) in the selective transfer hydrogenation is usually from 20:1 to 10000:1, preferably 50:1 to 1000:1. This ratio is related to Mol of substrate (which is compound of formula (I)) to mol of catalyst (compound of formula (III)).

Therefore, a further embodiment of the present invention is a transfer hydrogenation (TH¹¹), which is (TH), (TH¹), (TH²), (TH³), (TH⁴), (TH⁵), (TH⁶), (TH⁷), (TH⁸), (TH⁹) or (TH¹⁰), wherein substrate to catalyst ratio (s/c ratio) is from 20:1 to 10000:1, preferably from 50:1 to 1000:1.

The transfer hydrogenation according to the present invention allows the preparation of nerol/geraniol or ethyl nerol/ethyl geraniol in excellent yield.

The nerol/geraniol or ethyl nerol/ethyl geraniol which is obtained by the transfer hydrogenation according to the present invention can be used in any application wherein such fragrance compounds are used.

The invention is illustrated by the following Examples. All temperatures are given in °C and all parts and percentages are related to the weight.

### Examples

### Example 1: Production of the catalysts

In a 25 mL 2-necked round bottomed flask equipped with thermometer and a magnetic stirrer, 0.020 mmol of metal complex (M(Y)X₂]₂) and 0.044 mmol of a ligand (Z) were dissolved in 9 mL solvent at room temperature. The mixture was stirred for 30 min.

The solvent was ethyl acetate, isopropanol or methanol.

All of the following catalysts [(C1) - (C8)] were produced according to the process of Example 1

### Catalyst 1 (C1)

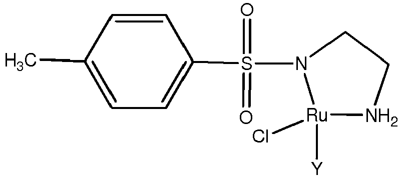

with

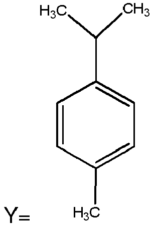

### Catalyst 2 (C2)

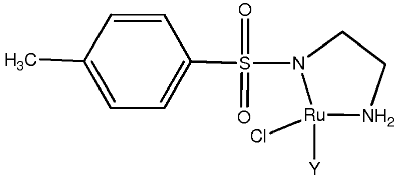

with

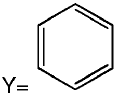

### Catalyst 3 (C3)

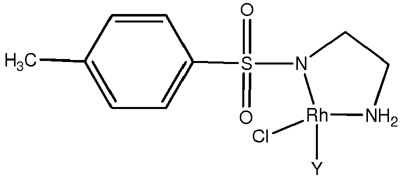

with

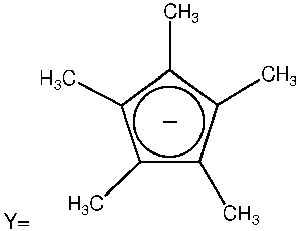

### Catalyst 4 (C4)

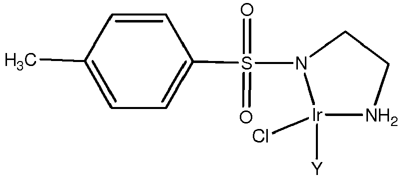

with

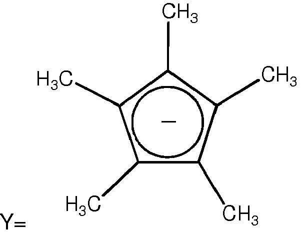

### Catalyst 5 (C5)

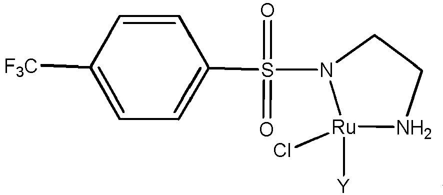

with

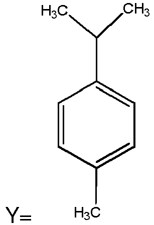

### Catalyst 6 (C6)

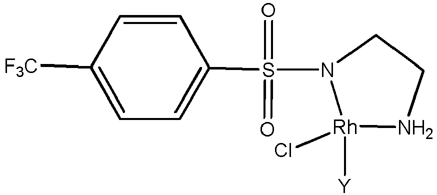

with

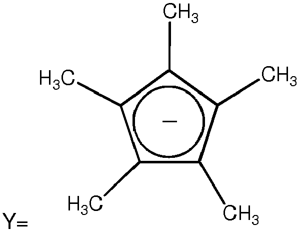

### Catalyst 7 (C7)

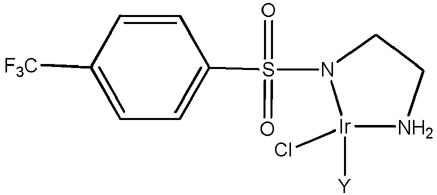

with

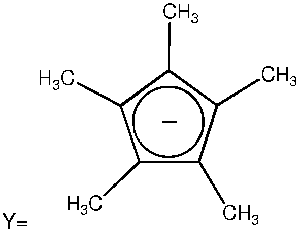

### Catalyst 8 (C8)

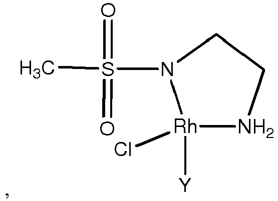

with

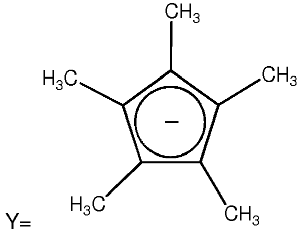

### Example 2: Selective transfer hydrogenation of Citral

To the solution (obtained according to Example 1) 6 mL solvent, 4 mmol (621 mg=700 µL) citral and 20 mmol reducing agent were added and stirred at the desired temperature (for all the following hydrogenation room temperature = 23°C was used)..
The reaction mixture was concentrated at 100 mbar by 40 °C. The residue was filtered over 10 g SiO₂ and washed with 50 mL ethyl acetate. The solution was concentrated at 100 mbar, 40 °C and the residue analyzed by gas chromatography with internal standard.

In the following table the results of the transfer hydrogenation with the catalysts of Example 1 are summarized.

| **Exp.** | **Cat.** | **Red.Agent** | **Solvent** | **Conversion [%]** | **Yield [%]** |
|---|---|---|---|---|---|
| **2a** | C1 | HCOOH/Et₃N (5:2) | Ethyl acetate | 99 | 97 |
| **2b** | C1 | i-propanol/KOH | i-propanol | 98 | 87 |
| **2c** | C3 | HCOOH/Et₃N (1:1) | Ethyl acetate | 99 | 99 |
| **2d** | C3 | HCOONa | Methanol | 99 | 83 |
| **2e** | C4 | HCOOH/Et₃N (1:1) | Ethyl acetate | 99 | 99 |
| **2f** | C5 | HCOOH/Et₃N (1:1) | Ethyl acetate | 86 | 86 |
| **2g** | C6 | HCOOH/Et₃N (1:1) | Ethyl acetate | 99 | 99 |
| **2h** | C7 | HCOOH/Et₃N (1:1) | Ethyl acetate | 99 | 99 |
| **2i** | C8 | HCOOH/Et₃N (1:1) | Ethyl acetate | 96 | 95 |

### Examples 3 and 4

In the following examples the catalyst is formed in situ. The following ligands have been used to form the catalyst in situ. The numbering of the ligands corresponds to the Examples they are used in.

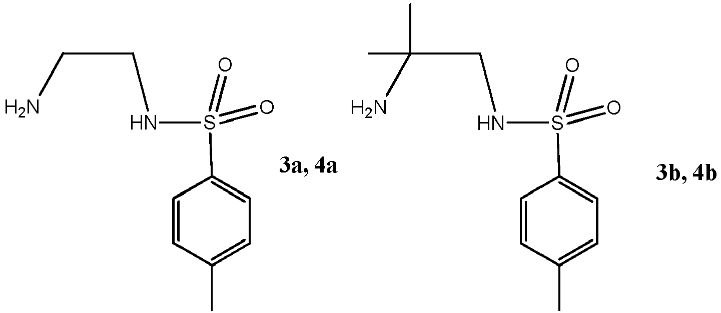

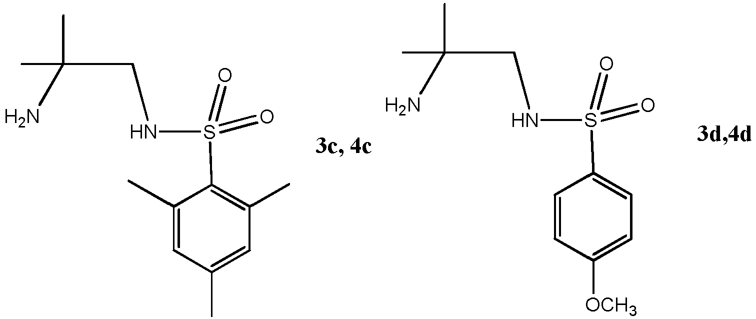

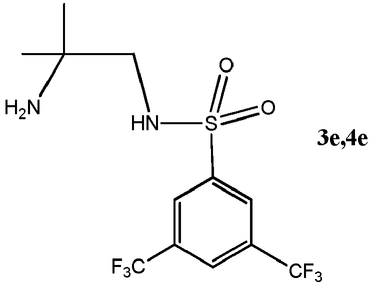

### Examples 3a - 3e: Transfer hydrogenation of citral (to nerol/geraniol); in situ formation of the catalyst

9 mg of dichloro(p-cymene)ruthenium(II) dimer (CAS No 52462-29-0) and the ligand (see table for the amount; the numbering of the Example corresponds to the numbering of the ligand) were dissolved in 3 ml of ethyl acetate. After stirring for 5 minutes, a solution of 500 mg citral in 2 ml ethyl acetate was added. The mixture was stirred for an additional 5 minutes and afterwards 1.30 ml of a 5:2 mixture of formic acid:triethylamine was added and the reaction mixture was stirred at room temperature. After 20 hours, the reaction mixture was analysed by HPLC. For the results (in regard to the conversion and the yield) see the table below.

| **Exp.** | **Amount of ligand** | **Conversion [%]** | **Yield [%]** |
|---|---|---|---|
| **3a** | 8 mg | 98.5 | 96.6 |
| **3b** | 9 mg | 98.2 | 94.0 |
| **3c** | 10 mg | 98.3 | 95.2 |
| **3d** | 9 mg | 98.5 | 95.9 |
| **3e** | 10 mg | 97.1 | 93.6 |

### Examples 4a - 4e: Transfer hydrogenation of ethyl citral (to ethyl nerol/ethyl geraniol); in situ formation of the catalyst

9 mg of Dichloro(p-cymene)ruthenium(II) dimer (CAS No 52462-29-0) and the ligand (see table for the amount; the numbering of the Example corresponds to the numbering of the ligand) were dissolved in 3 ml of ethyl acetate.

After stirring for 5 minutes, a solution of 530 mg of ethyl citral in 2 ml ethyl acetate was added.

The mixture was stirred for an additional 5 minutes and afterwards 13.0 ml of a 5:2 mixture of formic acid:triethylamine (5:2, Fluka, 1.30 ml) was added and the reaction mixture was stirred at room temperature. After 20 hours, the reaction mixture was analysed by HPLC. For the results (in regard to the conversion and the yield) see the table below.

| **Exp.** | **Amount of ligand** | **Conversion [%]** | **Yield [%]** |
|---|---|---|---|
| **4a** | 8 mg | 96.9 | 92.5 |
| **4b** | 9 mg | 99.1 | 95.1 |
| **4c** | 10 mg | 99.0 | 94.7 |
| **4d** | 9 mg | 99.2 | 95.1 |
| **4e** | 10 mg | 98.8 | 93.4 |

## Claims

1. A transfer hydrogenation of a compound of formula (I) 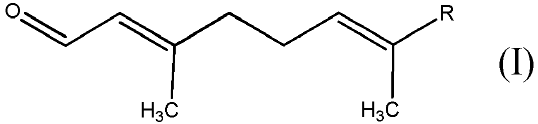 to a compound of formula (II) 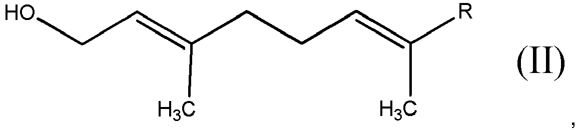 wherein R signifies -CH₃ or -CH₂CH₃,
**characterized in that** the hydrogenation is carried out in the presence of
a) an amino-amide-transition-metal catalyst of formula (III)
MX[Z-(-H)][Y] (III)
wherein
M is chosen from the group consisting of Ir, Rh and Ru, and
X signifies H or a halogen atom, and
Y is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
Z signifies a group of formula (IV) or (V) 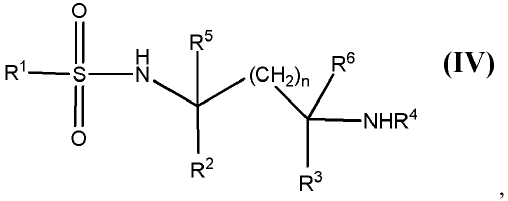 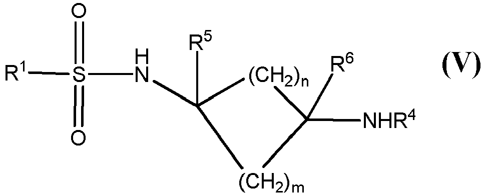
and the transition metal M is complexed to Z via the amine and the amide of Z; and
the monosulphonylated diamine Z is present in the complex as a monoanion and is accordingly denoted in formula III as
"Z-(-H)";
wherein
R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
R² and R³ each independently signify hydrogen, C₁-C₂-alkyl, C₄-C₇-cycloalkyl, or aryl which may be substituted, and
R⁴ signifies hydrogen or C₁-C₂-alkyl, and
R⁵ signifies hydrogen or C₁-C₂-alkyl, and
R⁶ signifies hydrogen or C₁-C₂-alkyl, and
n signifies 0,1,2 or 3, and
m signifies 0,1,2 or 3, and
with the proviso that in formula (V) the sum of n and m is 3 or 4, and
b) a H₂ donor, and
wherein the hydrogenation is carried out by using at least one solvent which also serves as hydrogen donor.

2. The transfer hydrogenation according to claim 1, wherein the hydrogenation is carried out in the absence of water.

3. The transfer hydrogenation according to claims 1 or 2, wherein the hydrogenation is carried out by using isopropanol as hydrogen donor and solvent.

4. The transfer hydrogenation according to anyone of the preceding claims, wherein the catalyst of formula (III)
M is chosen from the group consisting of Ir, Rh and Ru, and
X signifies H or Cl, and
Y is chosen from the group consisting of pentamethylcyclopentadienyl, benzene, p-cymene, toluene, anisole, xylene, 1,3,5-trimethylbenzene, p-dicyclohexylbenzene, naphthalene and tetralin, and
Z signifies a group of formula (IV) 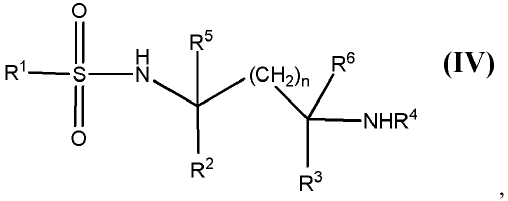
wherein
R¹ signifies C₁-C₂-alkyl which may be substituted with one or more fluorine atoms, C₂-C₆-alkenyl, C₂-C₆-alkynyl, C₄-C₇-cycloalkyl, aryl which may be substituted, heteroaryl, or camphor-10-yl, and
R² and R³ each independently signify hydrogen, C₁-C₂-alkyl, C₄-C₇-cycloalkyl, or aryl which may be substituted, and
R⁴ signifies hydrogen or C₁-C₂-alkyl, and
R⁵ signifies hydrogen or C₁-C₂-alkyl, and
R⁶ signifies hydrogen or C₁-C₂-alkyl, and
n signifies 0,1,2 or 3.

5. The transfer hydrogenation according to anyone of the preceding claims, wherein the catalyst is of formula (III') 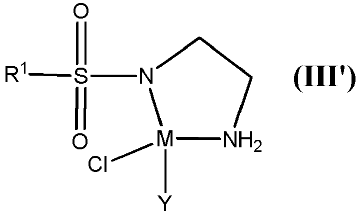 wherein
M is Ru, Rh or Ir, and
Y is an aromatic ligand chosen from the group consisting of 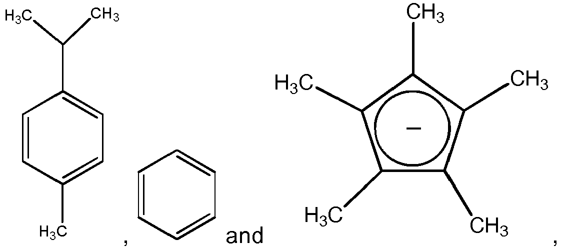 and
R¹ is -CH₃,

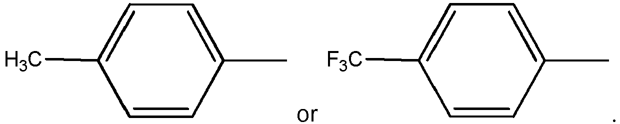

6. The transfer hydrogenation according to anyone of the preceding claims, wherein the catalyst is chosen from the group consisting of 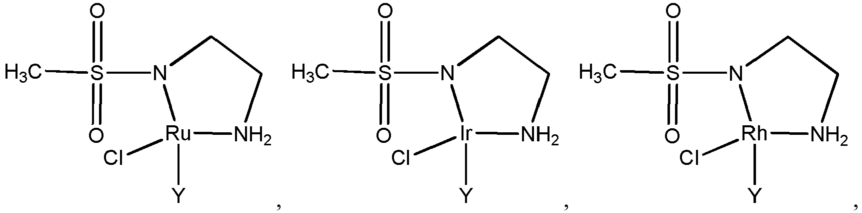 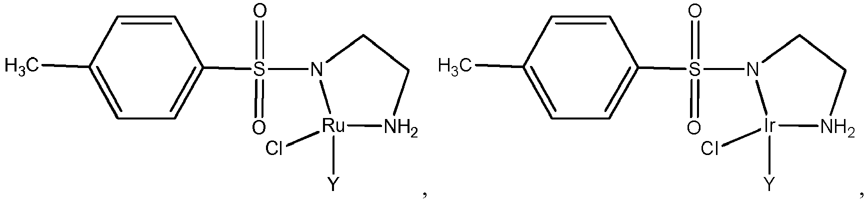 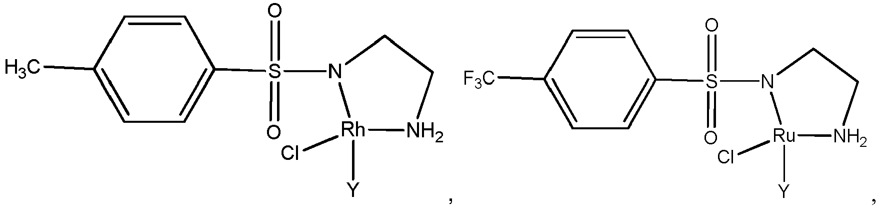 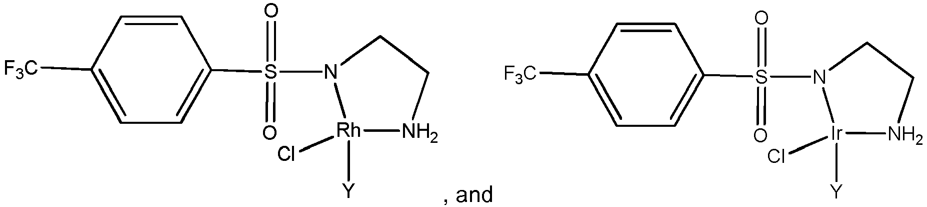 wherein Y is an aromatic ligand chosen from the group consisting of

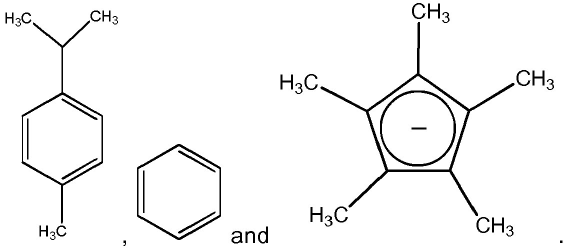

7. The transfer hydrogenation according to anyone of the preceding claims 1 - 5, wherein the catalyst is chosen from the group consisting of 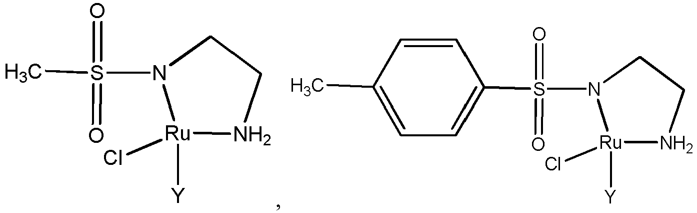 and 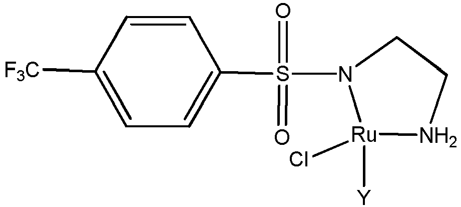 wherein Y is an aromatic ligand chosen from the group consisting of

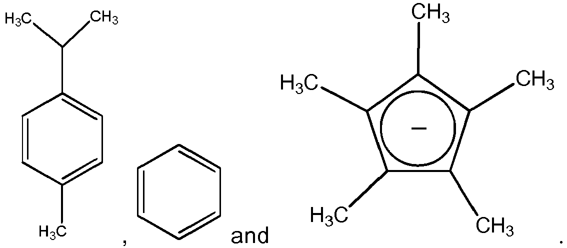

8. The transfer hydrogenation according to anyone of the preceding claims, wherein transfer hydrogenation reaction is carried out at a temperature of 0 - 100 °C.

9. The transfer hydrogenation according to anyone of the preceding claims, wherein the transfer hydrogenation reaction is carried out at a pressure of 50 - 1000 mbar.

10. The transfer hydrogenation according to anyone of the preceding claims, wherein the molar substrate to catalyst ratio (s/c ratio) is from 20:1 to 10000:1.

## Patentansprüche

1. Transferhydrierung einer Verbindung der Formel (I) 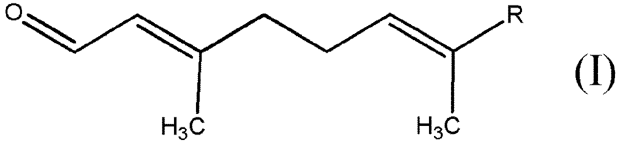 zu einer Verbindung der Formel (II), 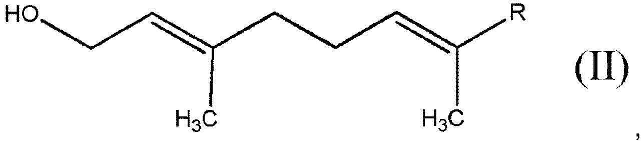 wobei R -CH₃ oder -CH₂CH₃ bedeutet,
**dadurch gekennzeichnet, dass** die Hydrierung in Gegenwart von
a) einem Amino-Amid-Übergangsmetallkatalysator der Formel (III),
MX[Z-(-H)][Y] (III)
wobei
M ausgewählt ist aus der Gruppe bestehend aus Ir, Rh und Ru und
X H oder ein Halogenatom bedeutet und
Y ausgewählt ist aus der Gruppe bestehend aus Pentamethylcyclopentadienyl, Benzol, p-Cymol, Toluol, Anisol, Xylol, 1,3,5-Trimethylbenzol, p-Dicyclohexylbenzol, Naphthalin und Tetralin und
Z eine Gruppe der Formel (IV) oder (V) bedeutet, 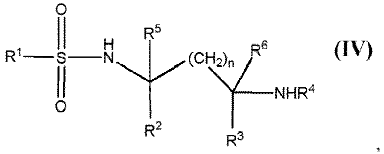 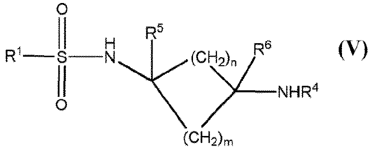
und das Übergangsmetall M über das Amin und das Amid von Z mit Z komplexiert ist; und
das monosulfonylierte Diamin Z in dem Komplex als Monoanion vorliegt und demgemäß in Formel III als" Z-(-H)" angegeben ist; wobei
R¹ C₁-C₂-Alkyl, das mit einem oder mehreren Fluoratomen substituiert sein kann, C₂-C₆-Alkenyl, C₂-C₆-Alkinyl, C₄-C₇-Cycloalkyl, Aryl, das substituiert sein kann, Heteroaryl oder Campher-10-yl bedeutet und
R² und R³ jeweils unabhängig Wasserstoff, C₁-C₂-Alkyl, C₄-C₇-Cycloalkyl oder Aryl, das substituiert sein kann, bedeuten und
R⁴ Wasserstoff oder C₁-C₂-Alkyl bedeutet und
R⁵ Wasserstoff oder C₁-C₂-Alkyl bedeutet und
R⁶ Wasserstoff oder C₁-C₂-Alkyl bedeutet und
n 0, 1, 2 oder 3 bedeutet und
m 0, 1, 2 oder 3 bedeutet und
mit der Maßgabe, dass in Formel (V) die Summe von n und m 3 oder 4 beträgt, und
b) einem H₂-Donator
durchgeführt wird, und wobei die Hydrierung unter Verwendung wenigstens eines Lösungsmittels, das auch als Wasserstoffdonator wirkt, durchgeführt wird.

2. Transferhydrierung gemäß Anspruch 1, wobei die Hydrierung ohne Vorhandensein von Wasser durchgeführt wird.

3. Transferhydrierung gemäß Anspruch 1 oder 2, wobei die Hydrierung unter Verwendung von Isopropanol als Wasserstoffdonator und Lösungsmittel durchgeführt wird.

4. Transferhydrierung gemäß einem der vorstehenden Ansprüche, wobei bei dem Katalysator der Formel (III)
M ausgewählt ist aus der Gruppe bestehend aus Ir, Rh und Ru und
X H oder Cl bedeutet und
Y ausgewählt ist aus der Gruppe bestehend aus Pentamethylcyclopentadienyl, Benzol, p-Cymol, Toluol, Anisol, Xylol, 1,3,5-Trimethylbenzol, p-Dicyclohexylbenzol, Naphthalin und Tetralin und
Z eine Gruppe der Formel (IV) bedeutet, 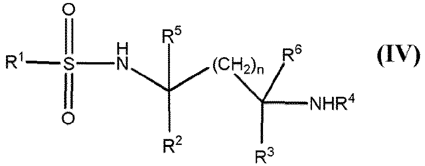
wobei
R¹ C₁-C₂-Alkyl, das mit einem oder mehreren Fluoratomen substituiert sein kann, C₂-C₆-Alkenyl, C₂-C₆-Alkinyl, C₄-C₇-Cycloalkyl, Aryl, das substituiert sein kann, Heteroaryl oder Campher-10-yl bedeutet und
R² und R³ jeweils unabhängig Wasserstoff, C₁-C₂-Alkyl, C₄-C₇-Cycloalkyl oder Aryl, das substituiert sein kann, bedeuten und
R⁴ Wasserstoff oder C₁-C₂-Alkyl bedeutet und
R⁵ Wasserstoff oder C₁-C₂-Alkyl bedeutet und
R⁶ Wasserstoff oder C₁-C₂-Alkyl bedeutet und
n 0, 1, 2 oder 3 bedeutet.

5. Transferhydrierung gemäß einem der vorstehenden Ansprüche, wobei der Katalysator von Formel (III') ist, 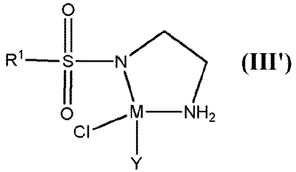 wobei
M Ru, Rh oder Ir ist und
Y ein aromatischer Ligand ausgewählt aus der Gruppe bestehend aus 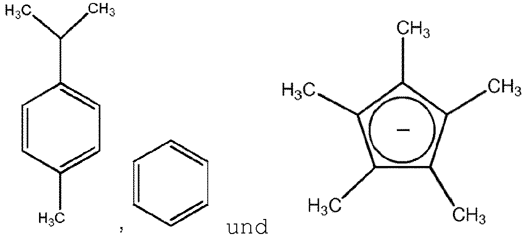 ist und
R₁ -CH₃, 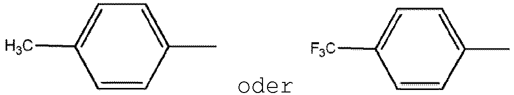 ist.

6. Transferhydrierung gemäß einem der vorstehenden Ansprüche, wobei der Katalysator ausgewählt ist aus der Gruppe bestehend aus 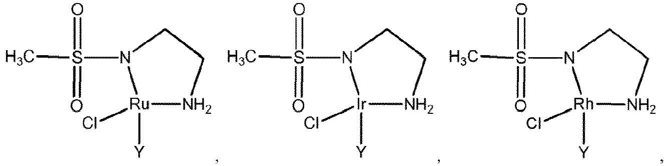 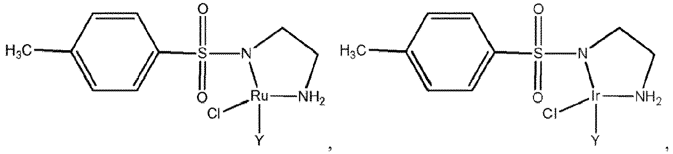 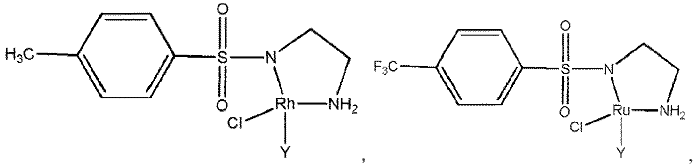 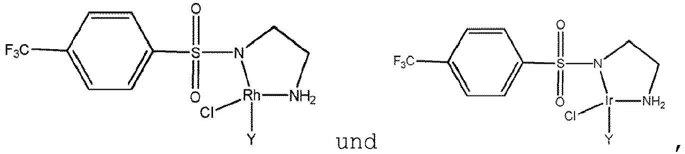 wobei Y ein aromatischer Ligand ausgewählt aus der Gruppe bestehend aus 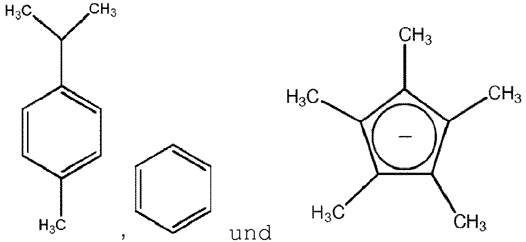 ist.

7. Transferhydrierung gemäß einem der vorstehenden Ansprüche 1-5, wobei der Katalysator ausgewählt ist aus der Gruppe bestehend aus 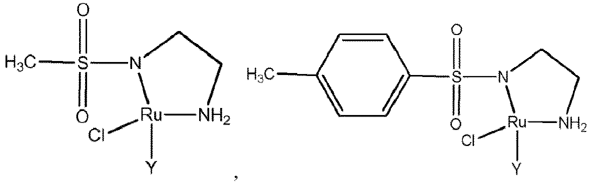 und 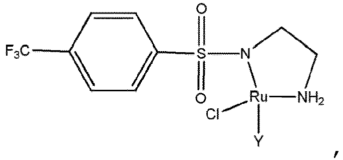 wobei Y ein aromatischer Ligand ausgewählt aus der Gruppe bestehend aus 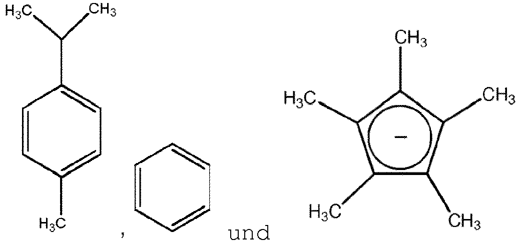 ist.

8. Transferhydrierung gemäß einem der vorstehenden Ansprüche, wobei die Transferhydrierungsreaktion bei einer Temperatur von 0-100 °C durchgeführt wird.

9. Transferhydrierung gemäß einem der vorstehenden Ansprüche, wobei die Transferhydrierungsreaktion bei einem Druck von 50-1000 mbar durchgeführt wird.

10. Transferhydrierung gemäß einem der vorstehenden Ansprüche, wobei das Molverhältnis von Substrat zu Katalysator (s/c-Verhältnis) von 20:1 bis 10000:1 beträgt.

## Revendications

1. Hydrogénation par transfert d'un composé de formule (I) 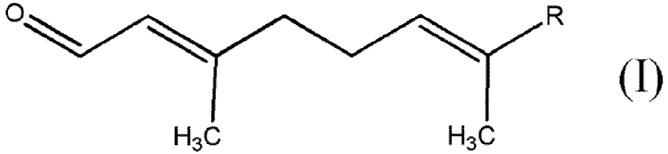 en un composé de formule (II) 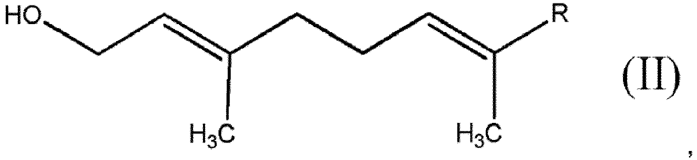 dans lequel R désigne -CH₃ ou -CH₂CH₃,
**caractérisée en ce que** l'hydrogénation est effectuée en présence de
a) un catalyseur amino-amide-métal de transition de formule (III)
MX[Z-(-H)][Y] (III)
dans lequel
M est choisi dans le groupe constitué par Ir, Rh et Ru et
X désigne H ou un atome d'halogène et
Y est choisi dans le groupe constitué par le groupe pentaméthylcyclopentadiényle, le benzène, le p-cymène, le toluène, l'anisole, le xylène, le 1,3,5-triméthylbenzène, le p-dicyclohexylbenzène, le naphtalène et la tétraline et
Z désigne un groupe de formule (IV) ou (V) 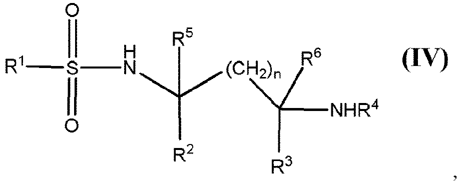 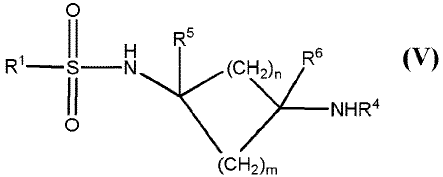
et le métal de transition M est complexé à Z par l'intermédiaire de l'amine et de l'amide de Z ; et la diamine monosulfonylée Z est présente dans le complexe sous forme d'un monoanion et est par conséquent représentée par « Z-(-H) » dans la formule (III) ;
dans lequel
R¹ désigne un groupe alkyle en C₁-C₂ qui peut être substitué par un ou plusieurs atomes de fluor, alcényle en C₂-C₆, alcynyle en C₂-C₆, cycloalkyle en C₄-C₇, aryle qui peut être substitué, hétéroaryle ou camphor-10-yle et
R² et R³ désignent chacun indépendamment l'atome d'hydrogène ou un groupe alkyle en C₁-C₂, cycloalkyle en C₄-C₇ ou aryle qui peut être substitué et
R⁴ désigne l'atome d'hydrogène ou un groupe alkyle en C₁-C₂ et
R⁵ désigne l'atome d'hydrogène ou un groupe alkyle en C₁-C₂ et
R⁶ désigne l'atome d'hydrogène ou un groupe alkyle en C₁-C₂ et
n désigne 0, 1, 2 ou 3 et
m désigne 0, 1, 2 ou 3 et
à condition que dans la formule (V) la somme de n et m soit de 3 ou 4, et
b) un donneur de H₂,
et l'hydrogénation étant effectuée à l'aide d'au moins un solvant qui sert également de donneur d'hydrogène.

2. Hydrogénation par transfert selon la revendication 1, l'hydrogénation étant effectuée en l'absence d'eau.

3. Hydrogénation par transfert selon les revendications 1 ou 2, l'hydrogénation étant effectuée à l'aide d'isopropanol en tant que donneur d'hydrogène et solvant.

4. Hydrogénation par transfert selon l'une quelconque des revendications précédentes, dans laquelle dans le catalyseur de formule (III)
M est choisi dans le groupe constitué par Ir, Rh et Ru et
X désigne H ou Cl et
Y est choisi dans le groupe constitué par le groupe pentaméthylcyclopentadiényle, le benzène, le p-cymène, le toluène, l'anisole, le xylène, le 1,3,5-triméthylbenzène, le p-dicyclohexylbenzène, le naphtalène et la tétraline et
Z désigne un groupe de formule (IV) 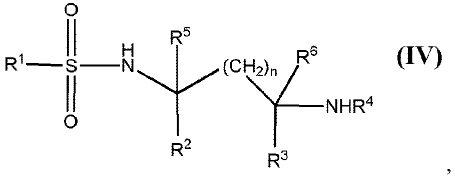
dans lequel
R¹ désigne un groupe alkyle en C₁-C₂ qui peut être substitué par un ou plusieurs atomes de fluor, alcényle en C₂-C₆, alcynyle en C₂-C₆, cycloalkyle en C₄-C₇, aryle qui peut être substitué, hétéroaryle ou camphor-10-yle et
R² et R³ désignent chacun indépendamment l'atome d'hydrogène ou un groupe alkyle en C₁-C₂, cycloalkyle en C₄-C₇ ou aryle qui peut être substitué et
R⁴ désigne l'atome d'hydrogène ou un groupe alkyle en C₁-C₂ et
R⁵ désigne l'atome d'hydrogène ou un groupe alkyle en C₁-C₂ et
R⁶ désigne l'atome d'hydrogène ou un groupe alkyle en C₁-C₂ et
n désigne 0, 1, 2 ou 3.

5. Hydrogénation par transfert selon l'une quelconque des revendications précédentes, dans laquelle le catalyseur est de formule (III') 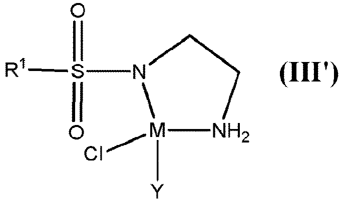 dans laquelle
M est Ru, Rh ou Ir et
Y est un ligand aromatique choisi dans le groupe constitué par 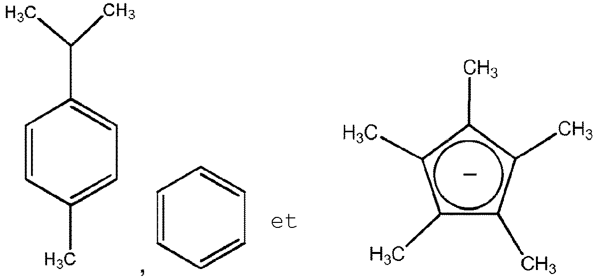
et
R¹ est -CH₃,

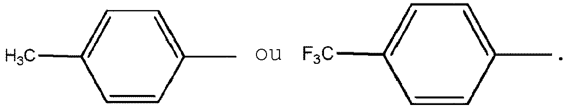

6. Hydrogénation par transfert selon l'une quelconque des revendications précédentes, dans laquelle le catalyseur est choisi dans le groupe constitué par 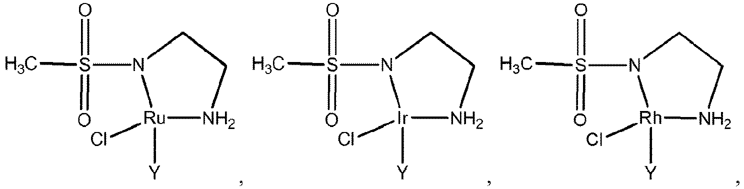 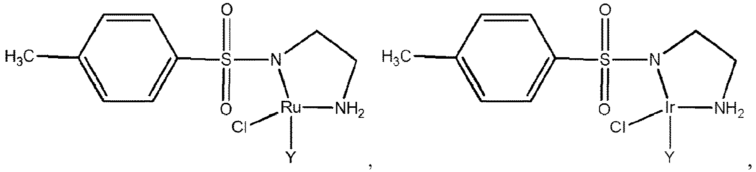 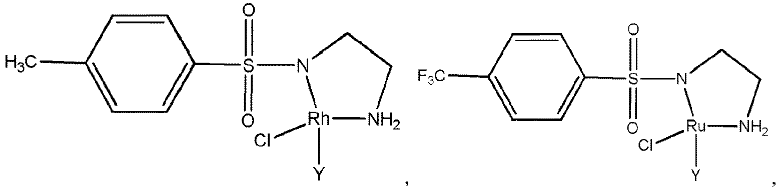 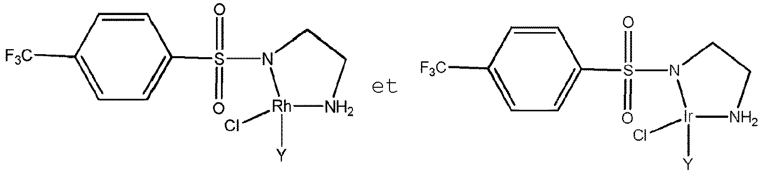 dans lesquels Y est un ligand aromatique choisi dans le groupe constitué par

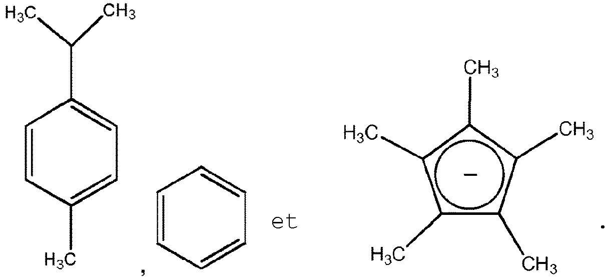

7. Hydrogénation par transfert selon l'une quelconque des revendications 1-5 précédentes, dans laquelle le catalyseur est choisi dans le groupe constitué par 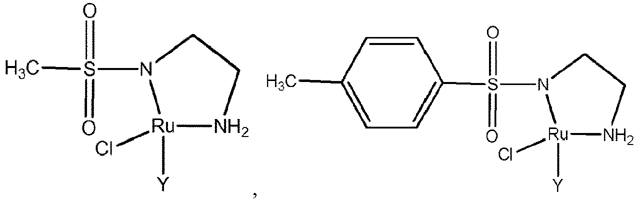 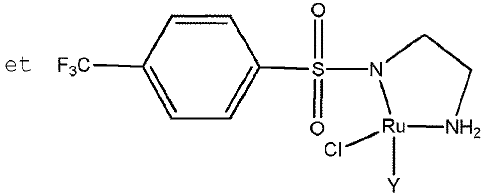 dans lesquels Y est un ligand aromatique choisi dans le groupe constitué par

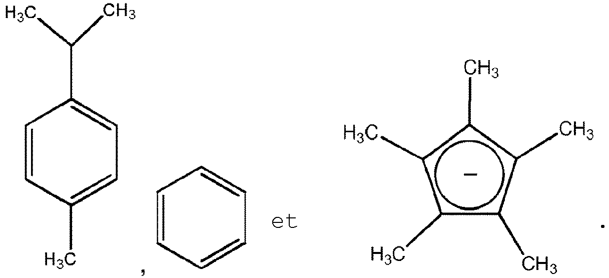

8. Hydrogénation par transfert selon l'une quelconque des revendications précédentes, dans laquelle la réaction d'hydrogénation par transfert est effectuée à une température de 0-100 °C.

9. Hydrogénation par transfert selon l'une quelconque des revendications précédentes, dans laquelle la réaction d'hydrogénation par transfert est effectuée à une pression de 50-1000 mbar.

10. Hydrogénation par transfert selon l'une quelconque des revendications précédentes, dans laquelle le rapport molaire du substrat au catalyseur (rapport s/c) est de 20:1 à 10000:1.
